# EUROPEAN PATENT APPLICATION

(11) **EP 3 918 978 A1**
(43) Date of publication of application: **08.12.2021**
(21) Application number: 20748197.9
(22) Date of filing: 10.01.2020
(51) Int. Cl.: A61B 5/00

(54) **WEARABLE DEVICE**

(30) Priority: 01.02.2019 CN 201910104517
(71) Applicant: New Chinese Biotechnology Corporation Ltd., Taipei City, 100, Taiwan (TW)
(72) Inventor: HUANG, Chien-Wen, Taipei City, Taiwan 100 (TW)
(74) Representative: Becker, Eberhard
(86) International application number: PCT/CN2020/071378
(87) International publication number: WO 2020/156099

(57) **Abstract**

Disclosed is a wearable device (100, 500, 600) for being worn on the torso of a user, at least one of limbs of the user or a combination thereof. The wearable device (100, 500, 600) comprises a wearable body (110, 210, 310, 410, 610) and at least one positioning unit (120, 520, 620), wherein the wearable body (110, 210, 310, 410, 610) has at least two fixing parts (111, 112, 113, 211, 212, 311, 312, 411) respectively fixed on at least two adjacent movable joints of the user, so that the wearable body (110, 210, 310, 410, 610) is fitted to the torso, the at least one of the limbs or the combination thereof; the wearable body (110, 210, 310, 410, 610) forms a positioning region (A1, A2, A3, A4) between the fixing parts (111, 112, 113, 211, 212, 311, 312, 411); a portion, between the two adjacent movable joints, of the torso, the at least one of the limbs or the combination thereof located is located in the positioning region (A1, A2, A3, A4); and the positioning unit (120, 520, 620) is configured in the positioning region (A1, A2, A3, A4) to correspond to a specific position of the user.

## Description

### BACKGROUND

### Technical Field

The disclosure relates to a wearable device.

### Description of Related Art

Along with development of medical technology, many convenient care products or medical devices are continuously developed in response to the needs of users. Among all these products and devices, a large number of wearable devices with a physiological signal monitoring function or capable of providing corresponding treatment methods have also been developed.

However, under consideration of versatility and accuracy, most of the existing wearable devices cannot be customized or even lightened in accordance with actual needs of the users. Therefore, the users are not allowed to receive required monitoring or treatment in a more comfortable manner.

For example, under the premise of monitoring accuracy, a wearable device usually has a complicated structure and is equipped with many electronic components, which may cause a burden on a user and the user may feel less comfortable. Further, if the weight of the wearable device is accordingly lightened, the device may not fit the body shape of the user appropriately, leading to displacement and inaccurate monitoring as a result. In other words, in the related art, no matter how, it is impossible for the wearable device to implement its functions smoothly and to optimize the user's sense of operation and comfort.

### SUMMARY

The disclosure is directed to a wearable device capable of defining a positioning region through fixing parts and providing a positioning unit in the positioning region, so as to smoothly provide a positioning effect to a specific position of a human body.

The disclosure provides a wearable device, which is adapted to be worn on at least one of a torso and limbs of a user or a combination thereof. The wearable device includes a wearable body and at least one positioning unit. The wearable body has at least two fixing parts fixed on at least two adjacent movable joints of the user, so that the wearable body fits on at least one of the torso, the limbs, or the combination thereof. The wearable body forms a positioning region between the fixing parts, and that a part of at least one of the torso, the limbs, or the combination thereof located between the at least two adjacent movable joints is within a range of the positioning region. The positioning unit is located in the positioning region to correspond to a specific position of the user.

In an embodiment of the disclosure, the fixing part has a thickened structure corresponding to a periphery of a convex part of the movable joint.

In an embodiment of the disclosure, the fixing part has an opening corresponding to a convex part of the movable joint.

In an embodiment of the disclosure, the fixing part has a thickened structure corresponding to a recess of the movable joint.

In an embodiment of the disclosure, the fixing part has a strap structure, which is tied to two opposite sides of the movable joint.

In an embodiment of the disclosure, the fixing part has a strap structure and a thickened structure, the strap structure is tied next to the movable joint, and the thickened structure corresponds to a bone bulge of the user.

In an embodiment of the disclosure, a material of the wearable body in the positioning region has elasticity.

In an embodiment of the disclosure, the positioning unit is physical coordinates arranged in the positioning region.

In an embodiment of the disclosure, the positioning unit is virtual coordinates arranged in the positioning region.

Based on the above description, in the wearable device, by configuring at least two fixing parts on the wearable body, when the wearable body is worn on at least one of the user's torso, limbs, or a combination thereof, the fixing parts may be fixed to at least two adjacent movable joints of the user. As such, the positioning region of the wearable body is defined between the fixing parts, and the positioning unit may correspond to the specific position of the user, so that relevant medical staff may provide medical means on the specific position of the user. In this way, the positioning region is fit to the torso of the user through restriction of the fixing parts, so that the wearable device that spans multiple movable joints may form an accurate positional corresponding relationship with the user.

To make the aforementioned more comprehensible, several embodiments accompanied with drawings are described in detail as follows.

### BRIEF DESCRIPTION OF THE DRAWINGS

FIG. 1 is a schematic view of a wearable device according to an embodiment of the disclosure.
FIG. 2A and FIG. 2B are partial schematic views of the wearable device of FIG. 1.
FIG. 3 is a schematic view of a wearable device according to another embodiment of the disclosure.
FIG. 4A to FIG. 4C are schematic views of the wearable device of FIG. 3 on different parts.
FIG. 5 is a schematic view of a wearable device according to another embodiment of the disclosure.
FIG. 6 is a partial schematic view of a wearable body of FIG. 5.
FIG. 7 is a schematic view of a wearable body according to another embodiment of the disclosure.
FIG. 8 is a partial schematic view of the wearable body of FIG. 7.
FIG. 9 is a schematic view of a wearable device according to another embodiment of the disclosure.
FIG. 10A and FIG. 10B are schematic views of a wearable device according to another embodiment of the disclosure.

### Reference Signs List

- 11:: acromioclavicular joint
- 12:: olecranon
- 13:: ulnar styloid
- 14:: radial styloid
- 15:: ischial tuberosity
- 16:: hip bone upper edge
- 17:: patella
- 18:: lateral malleolus
- 19:: medial malleolus
- 100, 500, 600:: wearable device
- 110, 210, 310, 410, 610:: wearable body
- 111, 112, 113, 211, 212, 3 11, 3 12, 411:: fixing part
- 112a, 211c, 212b, 212c, 312b, 411b, 411c:: opening
- 112b, 112c, 211d, 212a, 311b, 312a, 411a:: thickened structure
- 120, 520, 620:: positioning unit
- 130:: control unit
- 211 a, 211b, 311 a:: strap structure
- 630:: visual device
- A1, A2, A3, A4:: positioning region
- B1, B2, B3, B4, B5, B6:: non-positioning region

### DESCRIPTION OF THE EMBODIMENTS

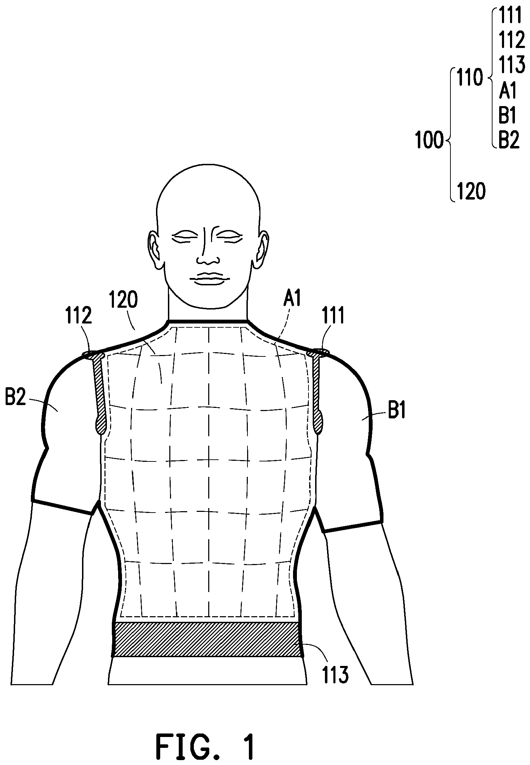

FIG. 1 is a schematic view of a wearable device according to an embodiment of the disclosure. Referring to FIG. 1, in this embodiment, a wearable device 100 includes a wearable body 110 and at least one positioning unit 120. The wearable body 110 is adapted to be worn on at least one of a torso (in this embodiment) and limbs of a user or a combination thereof. The positioning unit 120 is, for example, a coordinate grid, which is shown, configured or virtually indicated on the wearable body 110, and an outline or type of the positioning unit 120 is not limited by the disclosure. The positioning unit 120 of the embodiment is, for example, physical coordinates, which is, for example, a grid drawn on the wearable body 110. The positioning unit 120 is set at a specific place of the wearable body 110 to correspond to a specific position (for example, corresponding position of an acupuncture point or an internal organ) of the user. It should also be noted that the positioning unit 120 is fit to a body shape of the user along with the wearable body 110. In this way, after the user wears the wearable device 100, the relevant medical staff may learn a correspondence relationship between the positioning unit 120 and the specific position of the user, thereby further learning a physical state of the user, and especially the physical state corresponding to the specific position. For example, the medical staff may accordingly communicate with the user, and even learn a physiological signal of the user at the specific position, so as to facilitate providing corresponding treatment to the user. It should also be noted that the embodiment does not limit the number of the positioning units 120 and their combining method with the wearable body 110. In other embodiments, the positioning units 120 may also be appropriately integrated with the wearable body to form an integral structure, for example, when the wearable body is clothing used for being worn on human body, the positioning units 120 may be woven together with the wearable body.

Herein, taking a vest-type wearable body 110 as an example, it has a plurality of fixing parts 111-113, and the wearable body 110 is divided into a positioning region A1 located among the fixing parts 111-113 and non-positioning regions B1 and B2 located outside the fixing parts 111-113. It should be noted that the fixing parts 111-113 are fixed to adjacent movable joints (or diarthroses) of the user, and materials of the positioning region A1 and the non-positioning regions B1 and B2 have elasticity, and the positioning unit 120 is arranged in the positioning region A1 to correspond to the specific position of the user. The "movable joints" mentioned here refer to that connecting tissues between bones in the human body have gaps and lose continuity, such as shoulder joints, knee joints, etc., which are different from synarthroses and amphiarthroses, where the synarthroses are, for example, connections between skulls, and the amphiarthroses are, for example, connections between vertebra. In other words, the fixing parts 111-113 are fixed with movable joints, and the synarthroses and amphiarthroses are not positions where the fixing parts 111-113 need to be fixed with.

A reason of the above arrangement is that the wearable body 110 cannot form a connection relationship of the specific position with the user due to reasons such as different materials, manufacturing methods, or user's body shapes. For example, when users of different body shapes wear the same wearable body 110, the positioning unit 120 may be misaligned among these users, and cannot accurately correspond to the specific position of the user. Therefore, in this embodiment, as the wearable body 110 is provided with the plurality of fixing parts 111-113, and the fixing parts 111-113 are fixed on a plurality of movable joints of the user, especially the adjacent movable joints, the positioning region A1 may be defined among the fixing parts 111-113, and the positioning unit 120 is only arranged in a range of the positioning region A1.

In this way, once the wearable body 110 is worn on another user of a different body shape, the fixing parts 111-113 are still fixed at the same movable joints so that the positioning region A1 may still be maintained consistent, and meanwhile the material of the positioning region A1 is flexible, so that regarding the wearable body 110, once the fixing parts 111-113 are worn and fixed, the wearable body 110 may be fitted to the user (at least one of the torso, limbs, or a combination thereof, depending on a wearing position) at the positioning region A1, and more importantly, the positioning unit 120 in the positioning region A1 may smoothly correspond to the same specific position of the user, so as to avoid misalignment. In other words, the positioning region A1 is a range defined by a user's body margin and the fixing parts 111-113, and the range does not have a free end that allows the wearable body 110 to move arbitrarily. In addition, the embodiment does not limit a number and a form of the positioning units 120 or a configuration thereof in the positioning region A1, which may be appropriately changed according to actual usage conditions and requirements.

On the other hand, the non-positioning regions B1 and B2 shown in FIG. 1 only have the fixing parts 111 and 112 on single side, so that the non-positioning regions B1 and B2 will change a relative position relationship with the user due to reasons such as different materials, manufacturing methods or user's body shapes, i.e., they cannot always correspond to the specific location of the user. At this time, if the non-positioning regions B1 and B2 need to have the characteristics of the positioning region A1, i.e., the wearable body 110 is required to provide another fixing part at movable joints of the user's elbow, details thereof are to be described later.

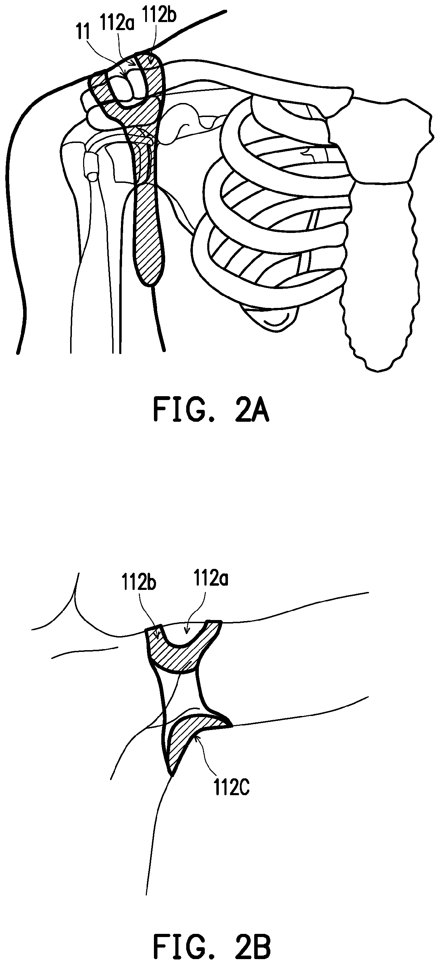

FIG. 2A and FIG. 2B are partial schematic views of the wearable device of FIG. 1. Corresponding human bones are additionally drawn to facilitate the description of the correspondence relationship with the fixing part, and the subsequent drawings will also be presented in a similar manner. Referring to FIG. 1, FIG. 2A and FIG. 2B together, the fixing parts 111 and 112 are fixed at shoulder joints of the user. The fixing part 112 is taken as an example herein for description, and since the fixing part 111 has the same structure, detailed description thereof is not repeated. In this embodiment, the fixing part 112 has an opening 112a and a thickened structure 112b. The opening 112a corresponds to a convex part of the shoulder joint, and the thickened structure 112b corresponds to a periphery of the convex part of the shoulder joint. Further, in order make the fixing part 112 to be smoothly fixed to the shoulder joint without movement caused by motions of the user, the opening 112a exposes an acromioclavicular joint 11, and at the same time, the flexible thickened structure 112b is used around the opening 112a to achieve a required fixing effect, and meanwhile increase the comfort of the user.

In another embodiment that is not shown, flexibility of the thickened structure may be adjusted by changing a material thereof, or an inflatable member may be used to provide a cushioning and positioning effect, thereby reducing the discomfort of the user when wearing for a long time.

In addition, as shown in FIG. 2B, since the fixing part 112 extends from the shoulder to the underarm, the fixing part 112 also has a thickened structure 112c for filling a recess of the shoulder joint, which is equivalent to an armpit of the user. In this way, the stability of the fixing part 112 fixed to the user's shoulder joint may be further strengthened.

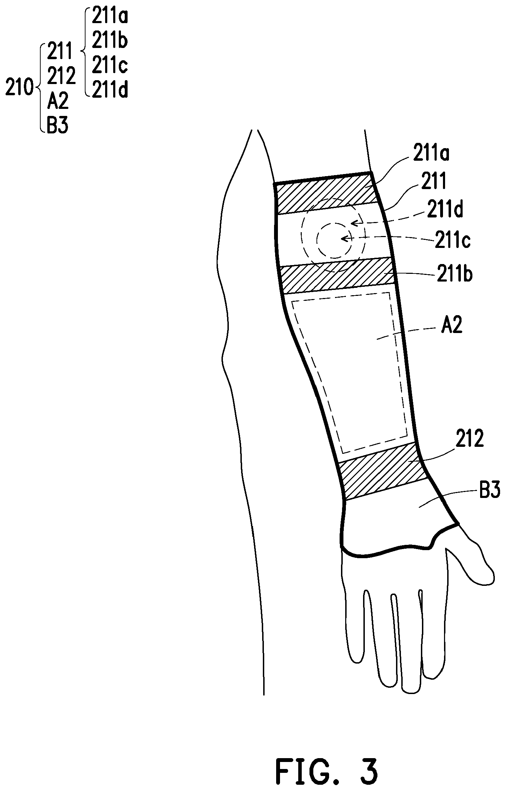

FIG. 3 is a schematic view of a wearable device according to another embodiment of the disclosure. A positioning region A2 is indicated, but the positioning unit 120 similar to that of the aforementioned embodiment is omitted, so that related features of a wearable body 210 may be well recognized. Referring to FIG. 3, in this embodiment, the wearable body 210 is adapted to be worn on an arm of the user, and the wearable body 210 includes fixing parts 211 and 212, the positioning region A2, and a non-positioning region B3. Similar to the aforementioned embodiment, the positioning region A2 needs to be defined (restricted) through the fixing parts 211 and 212 fixed to the adjacent movable joints of the arm.

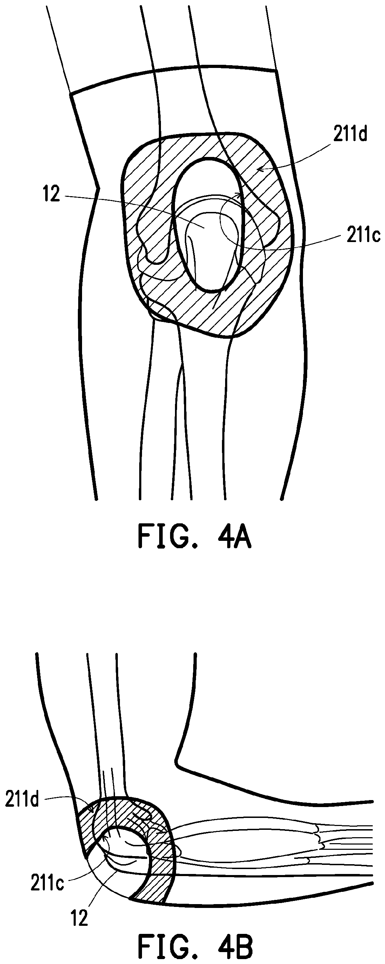

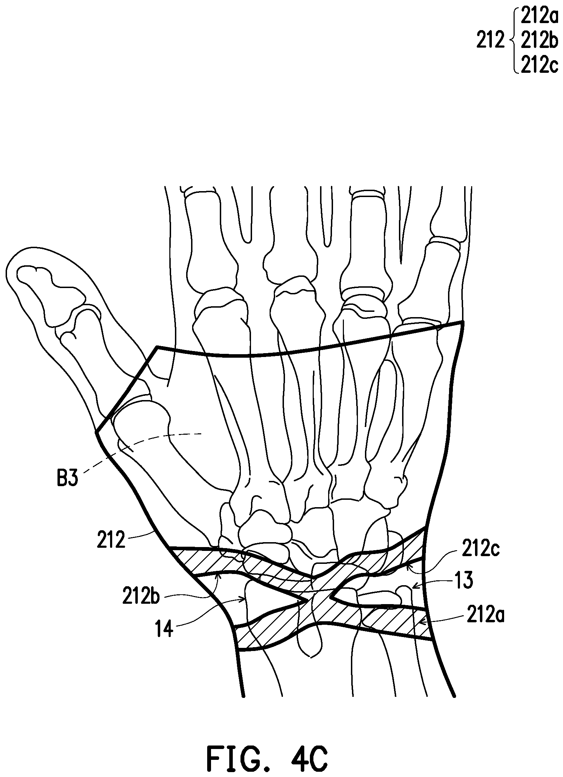

FIG. 4A to FIG. 4C are schematic views of the wearable device of FIG. 3 on different parts, in which FIG. 4A and FIG. 4B are corresponding schematic views of the fixing part 211 along with different arm movements of the user, and FIG. 4C is a partial schematic view of the fixing part 212 and the non-positioning region. Referring to FIG. 3, FIG. 4A, and FIG. 4B together, in this embodiment, the fixing part 211 includes strap structures 211a and 211b, which are tied to two opposite sides of an elbow joint along an extending direction of the arm, so that the fixing part 211 may be easily operated by the user to achieve a fixing effect. From this, it may also be seen that the two fixing parts 211, 212 of the wearable body 210 are respectively fixed on two adjacent movable joints (the elbow joint, a wrist joint) of the user, so that a limb section (i.e., a forearm) of the user between the joints may be located within the positioning region A2, and the positioning region A2 of the wearable body 210 may be fit to the forearm of the user. A form of the strap structure is not limited here, and the existing known straps, hook and loop fasteners, etc., may all be applied to the embodiment.

In addition, the fixing part 211 has a thickened structure 211d and an opening 211c, where the opening 211c is used to expose a convex part of the movable joint, and the thickened structure 211d corresponds to a periphery of the convex part of the movable joint. In detail, the thickened structure 211d corresponds to the periphery of an olecranon 12 to inner and outer epicondyles of the elbow joint, and is also made of the flexible material, which may improve the comfort of the user in addition to assisting in positioning. In FIG. 4C, the fixing part 212 fixed to the wrist joint of the user, and openings 212b and 212c are used to expose a convex part of the wrist joint, and a thickened structure 212a corresponds to the periphery of the convex part of the wrist joint. Further, the thickened structure 212a corresponds to a periphery of an ulnar styloid 13 and a radial styloid 14 of the wrist j oint, so that the ulnar styloid 13 is exposed from the opening 212c, and the radial styloid 14 is exposed from the opening 212b. In addition, the non-positioning region B3 is made of an elastic material, and extends from the fixing part 212 and opens to expose a purlicue, a palm, and fingers of the user for easy movement.

It should also be noted that, in another embodiment that is not shown, the wearable body may also include the entire arm of the user, i.e., the fixing part of the wearable device is only fixed on the shoulder and the wrist of the user, and the positioning region covers an upper arm and the forearm of the user.

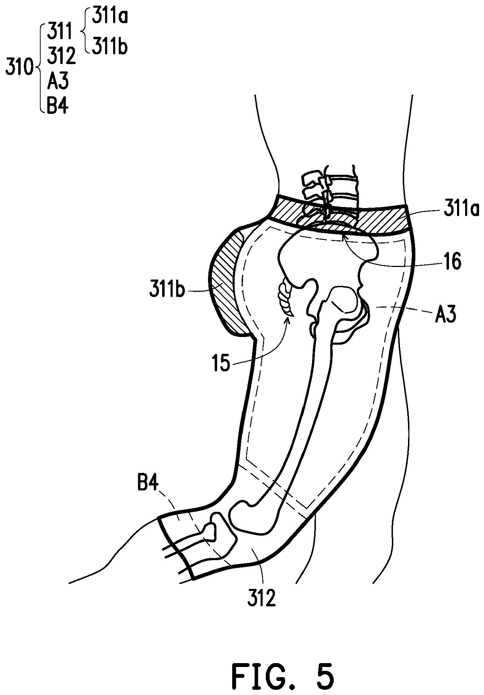

FIG. 5 is a schematic view of a wearable device according to another embodiment of the disclosure. Referring to FIG. 5, a wearable body 310 of the embodiment includes fixing parts 311 and 312, a positioning region A3, and a non-positioning region B4, where the fixing part 311 is fixed to a hip joint of the user, and the fixing part 312 is fixed to a knee joint of the user, so that the positioning region A3 between the fixing parts 311 and 312 is accordingly defined. The fixing part 311 has a strap structure 311a for being tied around the hip joint. Furthermore, the fixing part 311 further includes a thickened structure 311b, which corresponds to a bone bulge of the user. Furthermore, the fixing part 311 has the strap structure 311a corresponding to a hip bone upper edge 16, and the thickened structure 311b corresponding to an ischial tuberosity 15.

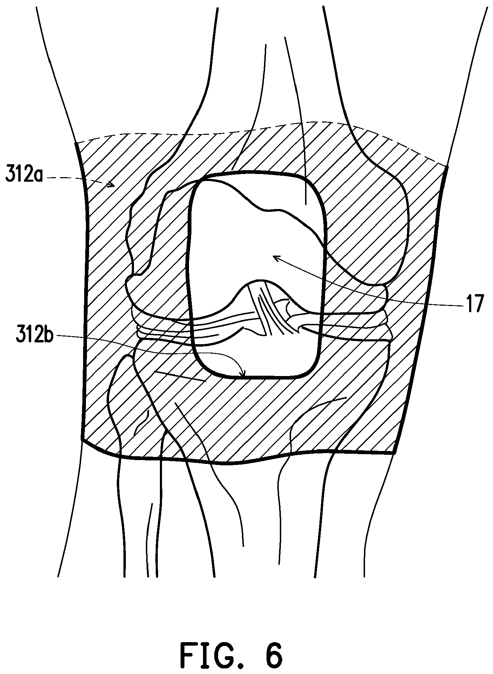

FIG. 6 is a partial schematic view of the wearable body of FIG. 5. Referring to FIG. 6, in this embodiment, the fixing part 312 has a thickened structure 312a and an opening 312b, where the opening 312b is used to expose the knee joint of the user, and the thickened structure 312a corresponds to a periphery of a convex part of the knee joint. Further, the thickened structure 312a is from the above of a patella 17 to medial and lateral femoral epicondyle, and from below the patella 17 to tibia inner and outer tibial plateau, i.e., the opening 312b only exposes the outermost convex part of the patella 17.

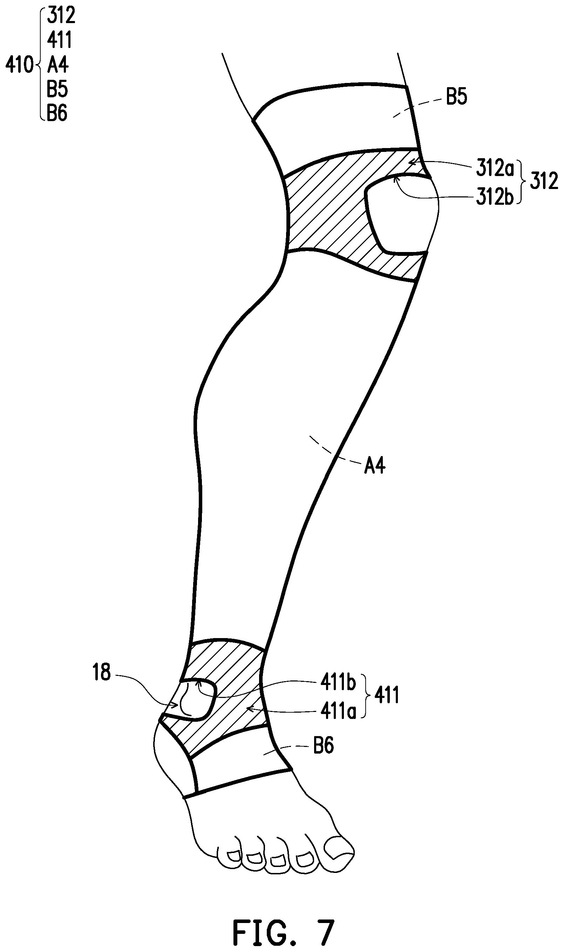

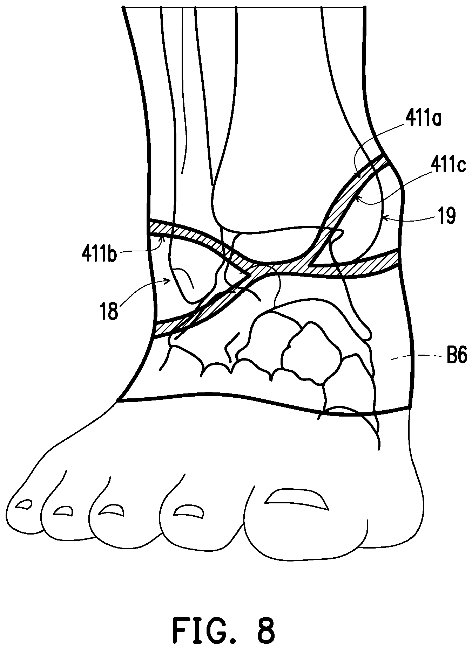

FIG. 7 is a schematic view of a wearable body according to another embodiment of the disclosure. FIG. 8 is a partial schematic view of the wearable body of FIG. 7. Referring to FIG. 7 and FIG. 8 together, it should be noted that a wearable body 410 includes fixing parts 312 and 411, a positioning region A4, and non-positioning regions B5 and B6. The fixing part 312 is as shown in FIG. 6, and detail thereof is not repeated. The positioning region A4 is located between the fixing parts 312 and 411 and restricted by the same, and corresponds to a calf of the user. The non-positioning regions B5 and B6 are respectively provided at two opposite ends of the wearable body 410.

In this embodiment, the fixing part 411 has openings 411b and 411c to expose a convex portion of an ankle joint of the user, and a thickened structure 411a corresponds to a periphery of the convex portion of the ankle joint. Furthermore, the thickened structure 411a corresponds to a periphery of a medial malleolus 19 of the ankle joint and a periphery of a lateral malleolus 18 of the ankle joint.

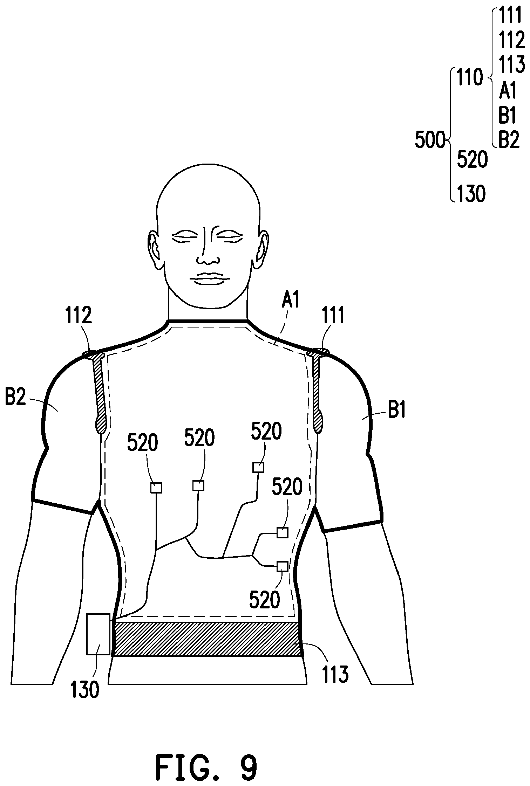

FIG. 9 is a schematic view of a wearable device according to another embodiment of the disclosure. Referring to FIG. 9, in addition to the same wearable body 110 as described above, different to the aforementioned embodiment, the wearable device 500 of this embodiment further includes positioning units 520 and a control unit 130. The positioning units 520 are, for example, electrode pads for corresponding specific positions of the user and are electrically connected to the control unit 130, so that the control unit 130 may obtain physiological signals of the specific positions of the user's body through the positioning units 520. In contrast, after the control unit 130 is electrically connected to an external treatment device (not shown), the positioning units 520 may also provide electrical signals to the specific positions of the body to achieve a treatment effect. In addition, similar to the aforementioned positioning unit 120, the positioning units 520 and the control unit 130 of the embodiment may also be woven together with the wearable body 110.

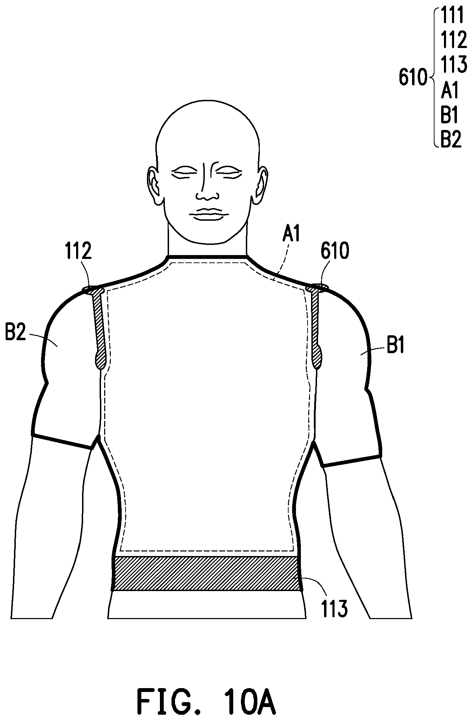

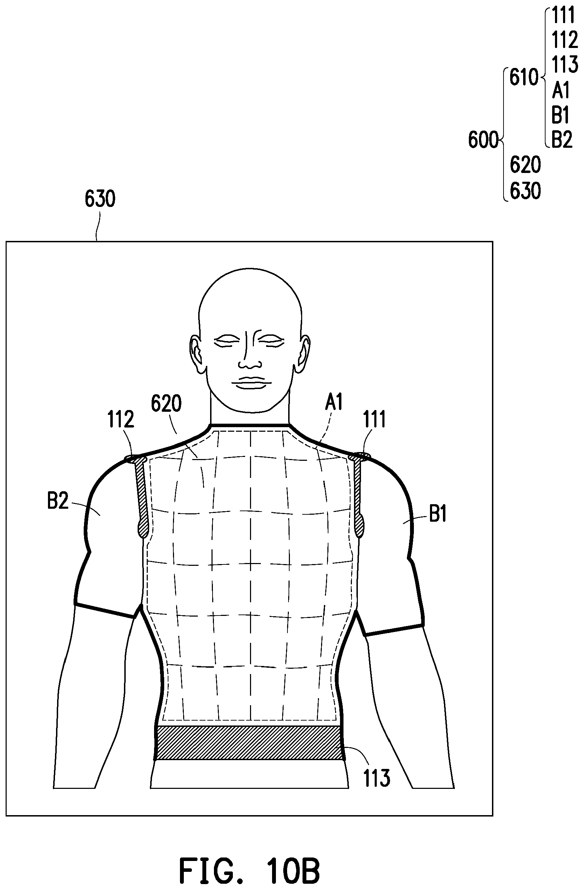

FIG. 10A and FIG. 10B are schematic views of a wearable device according to another embodiment of the disclosure. Referring to FIG. 10A and FIG. 10B together, different from the previous embodiment, the wearable device 600 of this embodiment includes a wearable body 610, a positioning unit 620, and a visual device 630. The positioning unit 620 is virtual coordinates displayed on the wearable body 610 through the visual device 630. The visual device 630 is, for example, a virtual reality (VR) device or an augmented reality (AR) device, or may be glasses with coordinates or a device with a light projection function, no matter how, coordinates may be visually generated on the wearable body 610 to correspond to specific positions of a wearer.

In summary, in the wearable device of the embodiments of the disclosure, by configuring at least two fixing parts on the wearable body, when the wearable body is worn on at least one of the user's torso, limbs, or a combination thereof, the fixing parts may be fixed to the movable joints of the user, such that the positioning region of the wearable body is defined between the fixing parts. In this way, the positioning region is fit to the torso of the user through restriction of the fixing parts, so that the wearable device that spans multiple movable joints may form an accurate positional corresponding relationship with the user.

In an embodiment, the fixing part has a thickened structure to correspond to the periphery of the convex part of the movable joint of the user. In another embodiment, the fixing part is arranged at the periphery of the convex part of the movable joint through a strap structure. In another embodiment, the fixing part has an opening for exposing the convex part of the movable joint of the user. In one of the above applications or a combination thereof, the stability of the fixing part at the convex part of the movable joint may be accordingly improved.

In another embodiment, after the required positioning units arranged in the positioning region are electrically connected to the control unit, the positioning units may be able to correspond to the specific positions of the user. As such, the control unit may provide or receive electrical signals to/from the specific positions of the user to achieve sensing and treatment effects.

It will be apparent to those skilled in the art that various modifications and variations can be made to the disclosed embodiments without departing from the scope or spirit of the disclosure. In view of the foregoing, it is intended that the disclosure covers modifications and variations provided they fall within the scope of the following claims and their equivalents.

## Claims

1. A wearable device, adapted to be worn on at least one of a torso and limbs of a user or a combination thereof, the wearable device comprising:
a wearable body, having at least two fixing parts fixed on at least two adjacent movable joints of the user, such that the wearable body fits on at least one of the torso, the limbs, or the combination thereof, wherein the wearable body forms a positioning region between the at least two fixing parts, and that a part of at least one of the torso, the limbs, or the combination thereof located between the at least two adjacent movable j oints is within a range of the positioning region; and
at least one positioning unit, located in the positioning region to correspond to a specific position of the user, wherein the positioning unit is physical coordinates or virtual coordinates arranged in the positioning region.

2. The wearable device according to claim 1, wherein the fixing part has a thickened structure corresponding to a periphery of a convex part of the movable joint.

3. The wearable device according to claim 1, wherein the fixing part has an opening corresponding to a convex part of the movable joint.

4. The wearable device according to claim 1, wherein the fixing part has a thickened structure corresponding to a recess of the movable joint.

5. The wearable device according to claim 1, wherein the fixing part has a strap structure tied to two opposite sides of the movable joint.

6. The wearable device according to claim 1, wherein the fixing part has a strap structure and a thickened structure, the strap structure is tied next to the movable joint, and the thickened structure corresponds to a bone bulge of the user.

7. The wearable device according to claim 1, wherein a material of the wearable body in the positioning region has elasticity.
